# EUROPÄISCHE PATENTANMELDUNG

(11) **EP 0 924 189 A2**
(43) Veröffentlichungstag der Anmeldung: **23.06.1999**
(21) Anmeldenummer: 98123143.4
(22) Anmeldetag: 04.12.1998
(51) Int. Cl.: C07C 209/64

(54) **Herstellung von N-Phenyl-1-naphthylamin**

(30) Priorität: 17.12.1997 DE 19756146
(71) Anmelder: Bayer Aktiengesellschaft, 51368 Leverkusen (DE)
(72) Erfinder: Baumgartner, Hanspeter Dr., 47807 Krefeld (DE); Frohn, Lutz Dr., 40699 Erkrath (DE); Klausener, Alexander Dr., 50259 Pulheim (DE); Arndt, Frank Dr., 47800 Krefeld (DE)

(57) **Zusammenfassung**

N-Phenyl-1-naphthylamin kann durch Umsetzung von Anilin und 1-Naphthylamin in flüssiger Phase bei 100-400°C und bei einem Reaktionsdruck, der oberhalb des Normaldrucks liegt, hergestellt werden. Als Katalysatoren können beispielsweise Bor und Fluor enthaltende Katalysatoren, Mono- oder Polysulfonsäuren, Jod, PCl₃, PCl₅ oder POCl₃ eingesetzt werden.

## Beschreibung

Die vorliegende Erfindung betrifft ein Verfahren zur Herstellung von N-Phenyl-1-naphthylamin (Phenyl-α-naphthylamin) der Formel (I) 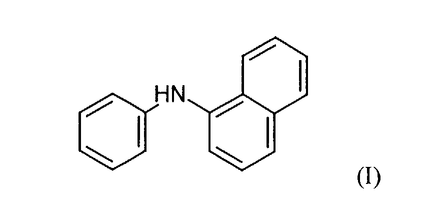 durch Umsetzung von Anilin mit 1-Naphthylamin, worin die Reaktion in Gegenwart eines Katalysators in kondensierter Phase und unter einem Druck oberhalb des Atmosphärendrucks durchgeführt wird.

N-Phenyl-1-naphthylamin wird beispielsweise als Alterungs- oder Oxidationsschutzmittel für Kautschuk sowie für Mineralölprodukte eingesetzt (Ullmanns Encyklopädie der technischen Chemie, 4. Auflage, Bd. 17, S. 107, Verlag Chemie, Weinheim 1979). Weitere Einsatzmöglichkeiten finden sich auf dem Gebiet der Farbstoffe. So erhält man etwa den Farbstoff Victoriablau, ein Diphenylnaphthylmethan-Derivat, ausgehend von N-Phenyl-1-naphthylamin (H. E. Fierz-David und L. Blangley, Farbenchemie 8. Aufl., S. 171, Springer-Verlag, Wien 1952). Auch Derivate des N-Phenyl- 1 -naphthylamins, die entweder aus dieser Verbindung selbst oder auf analoge Weise zu dieser Verbindung hergestellt werden, sind als Komponenten von Schmiermitteln von Interesse ( EP 716 141).

Nach H. E. Fierz-David und L. Blangley, Farbenchemie, 8. Aufl., S. 171, Springer-Verlag, Wien 1952, erhält man N-Phenyl-1-naphthylamin in Ausbeuten von 86-91 % der theoretischen Ausbeute durch Umsetzung von Anilin und 1-Naphthylamin bei Temperaturen von 195-215°C unter Normaldruck, wobei Sulfanilsäure als Katalysator dient. Das Verfahren benötigt lange Reaktionszeiten. Nach einer in DE-PS 241 853, sowie in, J. Prakt. Chem. 89, 1 (1914) beschriebenen Vorgehensweise erhält man N-Phenyl-1-naphthylamin durch Umsetzung von Anilin und 1-Naphthylamin bei Temperaturen von 225-250°C in Gegenwart katalytischer Mengen Jod unter Normaldruck. Die beschriebenen Reaktionsausbeuten sind deutlich geringer als im o.g. Verfahren, und auch die Reaktionszeiten sind unvorteilhaft lang. Nach J. Prakt. Chem. 89, 1 (1914) kann N-Phenyl-1-naphthylamin durch Umsetzung von 1-Naphthol mit Anilin bei Temperaturen zwischen 180 und 200°C ebenfalls in Gegenwart katalytischer Mengen Jod unter Normaldruck gewonnen werden. Die Ausbeuten erreichen jedoch nur 35-40 % der theoretischen Ausbeute, was diese Variante deutlich ungünstiger als die oben beschriebenen erscheinen läßt.

Allen genannten Verfahren ist gemeinsam, daß in ihrem Verlauf mehr oder weniger große Mengen unerwünschter Nebenprodukte, wie beispielsweise Diphenylamin, N-Phenyl-2-naphthylamin sowie 2-Naphthylamin gebildet werden. Vor allem die letztgenannten Verbindungen N-Phenyl-2-naphthylamin und 2-Naphthylamin können grundsätzlich durch Isomerisierung von bereits gebildetem N-Phenyl-1-naphthylamin bzw. nicht umgesetztem 1-Naphthylamin entstehen und zwar in Abhängigkeit von den zur Anwendung kommenden Reaktionsbedingungen und dem verwendeten Katalysator. Abgesehen davon, daß die Bildung derartiger unerwünschter Nebenprodukte aufgrund damit verbundener Materialverluste und dem erforderlich werdenden erhöhten trenntechnischen Aufwand zur Isolierung von reinem N-Phenyl-1-naphthylamin eine erhebliche Beeinträchtigung der Wirtschaftlichkeit des jeweiligen Herstellungsprozesses nach sich zieht, ist ferner zu berücksichtigen, daß insbesondere 2-Naphthylamin stark toxische Eigenschaften besitzt und seine Entstehung daher möglichst auszuschließen ist.

Es bestand somit die Aufgabe, ein wirtschaftliches Verfahren zur hochselektiven Herstellung von N-Phenyl-1-naphthylamin zu finden, bei dem die Bildung unerwünschter Nebenprodukte, vor allem aber die Bildung von 2-Naphthylamin, weitestgehend ausgeschlossen ist, das die Rückführung des zur Anwendung kommenden Katalysators erlaubt und das mit wirtschaftlich zufriedenstellenden Reaktionszeiten bzw. Raum-Zeit-Ausbeuten arbeitet.

Es wurde nun gefunden, daß sich N-Phenyl-1-naphthylamin in hohen Ausbeuten und ausgezeichneten Selektivitäten erhalten läßt, wenn man Anilin und 1-Naphthylamin in Gegenwart eines Katalysators unter einem Reaktionsdruck, der oberhalb des Normaldrucks liegt, miteinander umsetzt. Dies ist insofern überraschend, als Anilin bei höherem Druck mit sich selbst zu Diphenylamin reagiert. Weiterhin überraschend war die Beobachtung, daß einige erfindungsgemäß zum Einsatz kommenden Katalysatoren, nämlich solche mit einem Gehalt an Fluor und gegebenenfalls Bor, auf einfache Weise, also ohne Reinigung und Aufarbeitung, durch wäßrige Extraktion des ausreagierten Reaktionsgemisches wiedergewonnen werden können. Solchermaßen wiedergewonnene Katalysatoren können in die Reaktion zurückgeführt werden, ohne daß dies zu unakzeptablen negativen Folgen für Umsatz und Selektivität führen würde. Angesichts des oben geschilderten Standes der Technik konnten diese günstigen Ergebnisse nicht erwartet werden. Vielmehr hätte man bei der Umsetzung von Anilin und 1-Naphthylamin mit der Bildung größerer Mengen unerwünschter Nebenprodukte und einem Verlust des Katalysators nach Durchführung der Reaktion rechnen müssen. Insbesondere hätte man damit rechnen müssen, daß die Umsetzung von 1-Naphthylamin mit Anilin unter Druck zur Bildung signifikanter, die Wirtschaftlichkeit des Verfahrens erheblich beeinträchtigender Mengen Diphenylamin führt.

Es wurde ein Verfahren zur diskontinuierlichen oder kontinuierlichen Herstellung von N-Phenyl-1-naphthylamin durch Umsetzung von Anilin und 1-Naphthylamin in kondensierter Phase bei 100-400°C gefunden, das dadurch gekennzeichnet ist, daß man die Reaktion in Gegenwart eines Katalysators und bei einem Reaktionsdruck, der oberhalb des Normaldrucks liegt, durchführt.

Als Katalysatoren für das erfindungsgemäße Verfahren zur Herstellung von N-Phenyl-1-naphthylamin lassen sich grundsätzlich recht verschiedene Systeme einsetzen. Nachfolgend werden beispielhaft einige typische Katalysatorsysteme benannt.

### a) Fluor enthaltende Katalysatorsysteme

Es lassen sich beispielsweise entweder frisch hergestellte Katalysatoren oder wiedergewonnene Katalysatormengen, die im Zuge der Aufarbeitung von Reaktionsgemischen erhalten werden, verwenden. Grundsätzlich ist es auch möglich, Gemische aus wiedergewonnenem und frisch hergestelltem Katalysator zum Einsatz zu bringen. Als typischer Fluor enthaltender Katalysator für das erfindungsgemäße Verfahren wird im Falle des Einsatzes frischer Katalysatorchargen bevorzugt das Reaktionsprodukt verwendet, das bei der Umsetzung von Fluorwasserstoff, Borsäure und Anilin und/oder 1-Naphthylamin erhalten wird. Hierbei handelt es sich um ein salzartiges Produkt der Formel

[A] [B] (II),

in der
A für die Kationen [H]⁺, [NH₄]⁺, [Phenyl-NH₃]⁺, [(1-Naphthyl)-NH₃]⁺, [Phenyl-NH₂-Phenyl]⁺ oder für [Phenyl-NH₂-(1-Naphthyl)]⁺, bevorzugt für die Kationen [H]⁺, [NH₄]⁺, [Phenyl-NH₃]⁺ und [(1-Naphthyl)-NH₃]⁺ und besonders bevorzugt für die Kationen [NH₄]⁺ und [Phenyl-NH₃]⁺ steht und
B für Anionen des Typs [B(OH)ₙF₄₋ₙ]⁻, in der n ganzzahlige Werte von 0 bis 4 annehmen kann, und bevorzugt für das Anion [BF₄]⁻ steht.

Wichtige Einzelverbindungen der Formel (II) sind etwa [NH₄]⁺ [BF₄]⁻ und [Phenyl-NH₃]⁺[BF₄]⁻.

In der Praxis können die innerhalb der erfindungsgemäßen Reaktion wirksamen Katalysatoren Gemische verschiedener der allgemeinen Formel (II) entsprechenden Komponenten sein, deren Zusammensetzung sich jedoch nach Maßgabe der stöchiometrischen Verhältnisse sowie der Basizitäten und Dissoziationskonstanten der gegebenenfalls beteiligten kationischen Hauptkomponenten Wasser, Ammoniak, Anilin, 1-Naphthylamin und N-Phenyl-1-naphthyalmin einstellen und die im allgemeinen Anteile der Komponenten Wasser, Ammoniak, Anilin und/oder 1-Naphthylamin sowie gegebenenfalls noch weitere aromatische Diamine auch in unprotonierter Form enthalten. Diese Gemische liegen unter den Reaktionsbedingungen zur Herstellung von N-Phenyl-1-naphthylamin in homogen gelöster Form vor, wirken demnach also in ihrer Gesamtheit als homogenes Katalysatorsystem.

Die Herstellung von frischen Chargen des erfindungsgemäßen Katalysators erfolgt im allgemeinen unter Anwendung von dem Fachmann bekannten Verfahren. So ist es beispielsweise möglich, wäßrige Lösungen von Ammoniumtetrafluoroborat als Frischkatalysator einzusetzen. In einer bevorzugten Ausführungsform des erfindungsgemäßen Verfahrens wird die Herstellung frischer Chargen des erfindungsgemäßen Katalysators in räumlich getrennten, gesonderten Reaktionsgefäßen durchgeführt. Beispielsweise kann die Herstellung des Fluor enthaltenden Katalysators dadurch erfolgen, daß man als Reaktionskomponenten Anilin und/oder 1-Naphthylamin, Borsäure und Fluorwasserstoff miteinander zur Reaktion bringt. Bevorzugt werden Anilin, Borsäure und Fluorwasserstoff miteinander zur Reaktion gebracht. Für die Herstellung des Fluor enthaltenden Katalysators wird bevorzugt frisch zugeführtes Anilin und/oder 1-Naphthylamin eingesetzt. Es kann aber auch von Vorteil sein, hierfür bei der Aufarbeitung von ausreagierten Reaktionsgemischen anfallendes zurückgewonnenes Anilin und/oder 1-Naphthylamin einzusetzen.

Die für die Herstellung des Fluor enthaltenden Katalysators verwendete Borsäure wird bevorzugt als Feststoff eingesetzt. Grundsätzlich ist es aber auch möglich und kann gegebenenfalls von Vorteil sein, die Borsäure als Schmelze, als Lösung oder als Aufschlämmung, beispielsweise in Wasser und/oder Anilin, zum Einsatz zu bringen.

Der für die Herstellung des Fluor enthaltenden Katalysators verwendete Fluorwasserstoff wird im allgemeinen als Reinstoff oder als wäßrige Lösung zum Einsatz gebracht. In einer bevorzugten Ausführungsform setzt man den benötigten Fluorwasserstoff als konzentrierte wäßrige Lösung ein. Grundsätzlich sind aber auch andere Dosierweisen möglich, so beispielsweise die Einleitung von gasförmigem oder verflüssigtem reinen Fluorwasserstoff.

Zur Herstellung des Fluor enthaltenden Katalysators werden die beteiligten Komponenten im Gewichtsverhältnis Anilin und/oder 1-Naphthylamin : Borsäure : Fluorwasserstoff = (100 bis 2000) : (50 bis 250) : (50 bis 250) zum Einsatz gebracht. Bevorzugt bringt man Anilin, Borsäure und Fluorwasserstoff im Gewichtsverhältnis von von (300 bis 1000) : (100 bis 150) : (100 bis 150) zum Einsatz. Die Reihenfolge der Dosierung der genannten Komponenten ist grundsätzlich frei wählbar. In einer bevorzugten Ausführungsform wird zunächst Anilin vorgelegt, danach Borsäure hinzugefügt und schließlich wäßrige Fluorwasserstoffsäure eindosiert.

Die Herstellung des Fluor enthaltenden Katalysators erfolgt im allgemeinen bei einer Reaktionstemperatur zwischen 0 und 250°C, bevorzugt zwischen 30 und 200°C, besonders bevorzugt zwischen 70 und 150°C.

Bei der Herstellung des Fluor enthaltenden Katalysators kann es zweckmäßig sein zu rühren. Grundsätzlich ist es aber auch möglich, auf zusätzliches Rühren zu verzichten. Die im Verlaufe der Herstellung des Fluor enthaltenden Katalysators freiwerdende Reaktionswärme kann ganz oder teilweise durch äußere Kühlung abgeführt werden. Im allgemeinen erfolgt die Herstellung des Fluor enthaltenden Katalysators bei Normaldruck. Grundsätzlich ist es aber auch möglich, die Umsetzung bei erniedrigtem oder erhöhtem Druck durchzuführen. Der solchermaßen hergestellte Fluor enthaltenden Katalysator wird im allgemeinen in flüssiger Form ohne Isolierung oder weitere Aufreinigung in die Reaktion zur Herstellung von N-Phenyl-1-naphthylamin eingesetzt. Grundsätzlich ist es aber auch möglich, den Fluor enthaltenden Katalysator etwa unter reduziertem Druck zur Trockne einzudampfen und den dabei anfallenden festen Rückstand als katalytisch wirksames Gemisch in die Reaktion zur Herstellung von N-Phenyl-1-naphthylamin einzusetzen.

### b) Sulfonsäuren als Katalysatoren

Alternativ lassen sich im Rahmen des erfindungsgemäßen Verfahrens auch Mono-, Di- oder Trisulfonsäuren der Formel

R-(SO₃H)ₙ (III)

einsetzen, in der
R für geradkettiges oder verzweigtes C₁-C₂₀- Alkyl oder für unsubstituiertes oder mit bis zu 3 weiteren Substituenten substituiertes Phenyl, Naphthyl oder Pyridinyl steht, wobei als Substituenten Wasserstoff, geradkettiges oder verzweigtes C₁-C₆-Alkyl, Halogen, Amino, geradkettig oder verzweigtes C₁-C₆-Alkylamino oder Di-(C₁-C₆-alkyl)amino, Hydroxy, geradkettiges oder verzweigtes C₁-C₆-Alkyloxy oder Carboxyl in Frage kommen und
n die Zahl 1, 2 oder 3 bedeutet.

Bevorzugt werden Mono- und Disulfonsäuren der Formel (III) zum Einsatz gebracht, in der R für C₁-C₄-Alkyl oder für unsubstituiertes oder bis zu 2 weitere Substituenten tragendes Phenyl steht, wobei als Substituenten C₁-C₄-Alkyl, Fluor, Chlor, Brom, Amino oder Hydroxy in Frage kommen.

Besonders bevorzugt werden Monosulfonsäuren der Formel (III) zum Einsatz gebracht, in der R für Methyl oder Ethyl oder für unsubstituiertes oder einen weiteren Substituenten tragendes Phenyl steht, wobei als Substituent Methyl, Chlor, Brom, Amino, Hydroxy oder Carboxyl in Frage kommen.

Beispielhaft genannt seien Methan- und Ethansulfonsäure, Benzolsulfonsäure, p-Toluolsulfonsäure, 4-Chlorbenzolsulfonsäure, 4-Brombenzolsulfonsäure, 2-Sulfobenzoesäure, o-, m- und p-Aminobenzolsulfonsäure.

### c) Andere Katalysatoren

Grundsätzlich lassen sich im Rahmen des erfindungsgemäßen Verfahrens auch andere Katalysatoren einsetzen, die die Bildung von Diarylaminen aus Arylaminen katalysieren. Beispielhaft genannt seien Jod, Phosphortrichlorid, Phosphoroxychlorid und Phosphorpentachlorid. Im Falle der Verwendung hydrolyselabiler Katalysatoren muß jedoch auf eine Wiedergewinnung durch wäßrige Extraktion verzichtet werden.

Das erfindungsgemäße Verfahren beinhaltet zunächst die in der Flüssigphase innerhalb eines geeigneten Reaktors in Gegenwart des gewählten Katalysators ablaufende Umsetzung von Anilin mit 1-Naphthylamin unter einem Reaktionsdruck, der oberhalb des Normaldrucks liegt. Das nach Reaktionsende anfallende flüssige Produktgemisch wird mit Wasser und/oder im Falle eines Fluor enthaltenden Katalysators mit einer wäßrigen Lösung des gewählten Katalysators versetzt und in einer hierzu geeigneten Apparatur in eine überwiegend wäßrige und eine überwiegend organische flüssige Phase aufgetrennt.

Die den größten Teil des organischen Materials enthaltende flüssige Phase, die im wesentlichen das Reaktionsprodukt N-Phenyl-1-naphthylamin, nicht umgesetztes Ausgangsmaterial, also Anilin und 1-Naphthylamin, sowie kleine Anteile an Nebenprodukten enthält, wird abgetrennt und der weiteren Aufarbeitung zugeführt. Die den größten Teil des Katalysators enthaltende überwiegend wäßrige Phase wird ebenfalls abgetrennt. Sie kann ganz oder teilweise ausgeschleust werden und/oder im Falle eines Fluor enthaltenden Katalysators ganz oder teilweise ohne weitere Reinigung oder Aufarbeitung erneut als Katalysator für die Durchführung der erfindungsgemäßen Umsetzung von Anilin und 1-Naphthylamin Verwendung finden.

Die den größten Teil des organischen Materials enthaltende flüssige Phase wird gegebenenfalls noch ein oder mehrere weitere Male unter Zusatz von Wasser sowie gegebenenfalls eines oder mehrerer zusätzlicher anorganischer Hilfsstoffe gewaschen und danach der destillativen Aufarbeitung unterzogen. Bei der abschließenden destillativen Aufarbeitung des Reaktionsproduktes fällt N-Phenyl-1-naphthylamin in sehr reiner Form an. Überschüssiges Anilin und/oder 1-Naphthylamin wird gleichfalls in einer solchen Güte erhalten, daß sich beide Komponenten problemlos in den Herstellungsprozeß zurückführen lassen. Geringe Mengen Destillationsrückstände werden einer Reststoffverwertung zugeführt oder verworfen.

Die Durchführung des erfindungsgemäßen Verfahrens zur Herstellung von N-Phenyl-1-naphthylamin kann grundsätzlich in seiner Gesamtheit oder in Teilbereichen auf diskontinuierliche oder auf kontinuierliche Weise erfolgen. Bevorzugt erfolgt die eigentliche Umsetzung von Anilin mit 1-Naphthylamin auf diskontinuierliche Weise; die nachfolgende Aufarbeitung kann sich aber in kontinuierlicher Weise anschließen.

Die außerordentlich hohe Effektivität und Selektivität des erfindungsgemäßen Verfahrens, vor allem im Hinblick auf das Ausbleiben der unerwünschten Bildung inakzeptabel hoher Anteile an Diphenylamin, ist überraschend und wird in der Literatur nicht vorbeschrieben. Im Gegenteil wurden kompliziertere bzw. gegenüber Nebenreaktionen bedeutend störanfälligere Operationen wie beispielsweise die oben diskutierten Alternativverfahren vorgeschlagen.

Die zur Herstellung von N-Phenyl-1-naphthylamin eingebrachten Reaktanden Anilin und 1-Naphthylamin werden in einem molaren Verhältnis eingesetzt, das bei 1 : 3 bis 10 : 1, bevorzugt bei 1 : 2 bis 5 : 1 und besonders bevorzugt bei 1 : 1 bis 3 : 1 liegt. Dabei ist es belanglos, in welchem Aggregatzustand die jeweiligen Komponenten in die Reaktion eingebracht werden. In einer bevorzugte Ausführungsform werden sowohl Anilin als auch 1-Naphthylamin in flüssiger Form in die Reaktion eingebracht. Es kann aber auch von Vorteil sein, etwa 1-Naphthylamin als Feststoff oder Anilin in verdampfter Form in das Reaktionsgemisch einzubringen.

Eine besonders vorteilhafte Variante des erfindungsgemäßen Verfahrens zur Herstellung von N-Phenyl-1-naphthylamin, die ebenfalls Gegenstand der Erfindung ist, besteht darin, die Reaktionskomponente 1-Naphthylamin oder ein Gemisch der Reaktionskomponente 1-Naphthylamin mit einem Teil, etwa 5-40%, des zur Reaktion gebrachten Anilins nach Vorlegen der restlichen Menge der Reaktionskomponente Anilin sowie des gewählten Katalysators während oder nach Aufheizen auf die gewählte Reaktionstemperatur und nach Einstellung des gewählten Druckes, im Verlaufe eines bestimmten Zeitraumes in das Reaktionsgefäß einzudosieren.

Der entsprechend dem erfindungsgemäßen Verfahren in die Reaktion zur Herstellung von N-Phenyl-1-naphthylamin eingebrachte Katalysator bzw. das Katalysatorgemisch wird in einer solchen Menge eingesetzt, daß das molare Verhältnis zwischen der katalytisch wirksamen Komponente im Katalysator bzw. dem Katalysatorgemisch und der gesamten Stoffmenge des in die Reaktion eingesetzten Anilins und 1-Naphthylamins zwischen 1 : 10 und 1 : 100.000, bevorzugt zwischen 1 : 25 und 1 : 10.000 und besonders bevorzugt zwischen 1 : 50 und 1 : 1.000 liegt. Der erfindungsgemäße Katalysator bzw. das Katalysatorgemisch wird im allgemeinen in flüssiger oder fester Form, als Reinstoff, Lösung oder Anschlämmung in das Reaktionsgemisch eingebracht. In einer besonderen Ausführungsform wird im Falle eines Fluor enthaltenden Katalysators der Katalysator bzw. das Katalysatorgemisch nach Eindampfen katalysatorhaltiger Lösungen, wie sie etwa bei der Herstellung frischer Katalysatorchargen oder bei der Wiedergewinnung von Katalysator im Zuge der weiteren Aufarbeitung ausreagierter Reaktionsgemische anfallen, in fester Form als katalytisch wirksamer Feststoff oder katalytisch wirksames Feststoffgemisch in die erfindungsgemäße Reaktion zur Herstellung von N-Phenyl-1-naphthylamin eingebracht.

Die erfindungsgemäße Reaktion zur Herstellung von N-Phenyl-1-naphthylamin wird im Druckbereich oberhalb Normaldruck bis 15 bar, bevorzugt bei 1,5 bis 10 bar, besonders bevorzugt bei 1,5 bis 5 bar, durchgeführt. Die erfindungsgemäße Reaktion zur Herstellung von N-Phenyl-1-naphthylamin wird im Temperaturbereich zwischen 100 und 400°C, bevorzugt zwischen 150 und 350°C, besonders bevorzugt zwischen 180 und 300°C, durchgeführt. Zur Gewährleistung des gewählten Temperaturniveaus wird dem Reaktionsgefäß durch außen- und/oder innenliegende Heizaggregate die erforderliche Wärmeenergie zugeführt.

Das Reaktionsgut wird im Verlaufe der erfindungsgemäßen Reaktion zur Herstellung von N-Phenyl-1-naphthylamin durchmischt. Dies kann beispielsweise durch Rühraggregate, durch außen- oder innenliegende Umpumpvorrichtungen oder auf eine andere geeignete Weise geschehen. Grundsätzlich ist es aber auch möglich, im Verlaufe der Reaktion auf jegliche aktive Durchmischung des Reaktionsgutes zu verzichten, da das im Zuge der Umsetzung der Reaktanden freiwerdende Ammoniakgas sowie siedende Reaktionskomponenten, wie insbesondere Anilin, ebenfalls zu einer Durchmischung führen.

Das im Zuge der Umsetzung der Reaktanden entweichende Ammoniakgas wird abgetrennt und einer weiteren Verwertung oder einer geregelten Entsorgung zugeführt. Die Abtrennung des im Zuge der Umsetzung der Reaktanden entweichenden Ammoniakgases erfolgt im allgemeinen mit Hilfe dazu dem Fachmann bekannter geeigneter Kondensatoren, deren Betriebsbedingungen so gewählt werden, daß sie den Durchtritt des Ammoniakgases erlauben, die erheblich höher siedenden Reaktanden sowie die Reaktionsprodukte jedoch im Reaktionsbereich zurückhalten.

Neben den genannten gewählten Reaktionsbedingungen sowie den apparativen Parametern ist die zeitliche Dauer der erfindungsgemäßen Reaktion zur Herstellung von N-Phenyl-1-naphthylamin maßgeblich für den erzielten Umsetzungsgrad der eingesetzten Reaktanden Anilin und 1-Naphthylamin. Da das erfindungsgemäße Verfahren zur Herstellung von N-Phenyl-1-naphthylamin die Rückführung nicht umgesetzten Anilins und/oder 1-Naphthylamins ermöglicht, ist die Reaktionszeit in weiten Grenzen variierbar und kann unter prozeßökonomischen Gesichtspunkten beispielsweise so gewählt werden, daß die Raum-Zeit-Ausbeuten des Reaktionsteils des erfindungsgemäßen Verfahrens möglichst hoch und der trenntechnische Aufwand zur Rückführung nicht umgesetzten Ausgangsmaterials möglichst klein ist. Im allgemeinen werden mehr als 50 %, bevorzugt mehr als 60 %, besonders bevorzugt mehr als 70 % des bei diskontinuierlicher Verfahrensweise in einen diskreten Reaktionsansatz bzw. bei kontinuierlicher Verfahrensweise in die Reaktionszone eingebrachten 1-Naphthylamins umgesetzt.

Das nach Beendigung der Reaktion anfallende Gemisch wird, bevorzugt nach Abkühlen auf eine Temperatur unterhalb von 100°C, zunächst mit Wasser und/oder wasserhaltiger Lösung wiedergewonnenen Katalysatorgemisches aus vorangegangenen Aufarbeitungen versetzt und vermischt. Das dabei anfallende zweiphasige Gemisch wird einer Phasentrennung zugeführt. Die Menge des in diesen Extraktionsprozeß eingesetzten Wassers und/oder im Falle von Fluor enthaltenden Katalysatoren der wasserhaltigen Lösung des aus vorangegangenen Aufarbeitungen wiedergewonnenen Katalysatorgemisches wird vorzugsweise so gewählt, daß der nach der genannten Phasentrennung schließlich anfallende wäßrige Extrakt ca. 1-35% nicht verdampfbare katalytisch wirksame Komponenten enthält. Die Temperatur des zweiphasigen Gemisches wird im Verlauf der Extraktion und der Phasentrennung im allgemeinen im Bereich zwischen 40 und 100°C gehalten.

Die nach der genannten Phasentrennung erhaltene organische Phase wird vorzugsweise einer weiteren Extraktion unterworfen. Grundsätzlich ist es aber auch möglich, sie ohne weitere Behandlung aufzudestillieren. Die zweite Extraktion erfolgt mit Wasser, dem gegebenenfalls zwischen 0 und 50 %, bevorzugt zwischen 5 und 30 %, besonders bevorzugt zwischen 10 und 20 % eines oder mehrerer Hilfsstoffe, bevorzugt eines oder mehrerer salzartiger anorganischer Hilfsstoffe, besonders bevorzugt eines oder mehrerer Stoffe aus der Gruppe der Alkali- und Erdalkalimetallhalogenide, der Alkalimetallsulfate, der Alkalimetallcarbonate und der Alkalimetallhydrogencarbonate zugesetzt wurde. Die Temperatur des zweiphasigen Gemisches wird im Verlauf der Extraktion und der Phasentrennung im allgemeinen im Bereich zwischen 30 und 90°C gehalten. Für den Fall, daß Jod oder jodhaltige Katalysatorgemische zum Einsatz kommen, kann es sinnvoll sein, den zweiten Extraktionsschritt in Gegenwart wasserlöslicher salzartiger Verbindungen vorzunehmen, die in der Lage sind, Jodreste zu binden bzw. durch Reduktion zu wasserlöslichem Jodid umzuwandeln. Beispielhaft seien Natriumsulfit, Natriumbisulfit, Natriumthiosulfat oder Natriumdithionit genannt. Das bei der genannten zweiten Extraktion anfallende zweiphasige Gemisch wird einer Phasentrennung zugeführt. Die nach der Phasentrennung erhaltene wäßrige Phase wird zum Teil für weitere Extraktionen wiederverwendet, zum Teil ausgeschleust und einer Verwertung oder einer geregelten Entsorgung zugeführt. Die nach der Phasentrennung erhaltene organische Phase wird der destillativen Auftrennung zugeführt. Beispielsweise mit Hilfe konventioneller, dem Fachmann bekannter Destillationsapparaturen und Destillationstechniken wird diese destillative Auftrennung vorzugsweise so durchgeführt, daß in dem rohen Destillationsgemisch vorhandenes nicht umgesetztes Anilin und 1-Naphthylamin vollständig wiedergewonnen und in spätere Reaktionen zur Herstellung von N-Phenyl-1-naphthylamin wiedereingesetzt werden können.

### Beispiele

### Beispiel 1

In einen Hochdruckautoklaven aus V4A, der mit einem Intensivkühler und hinter dem Kühler mit einem Druckhalteventil ausgestattet war, legte man ein Gemisch aus 279,40 g (3,00 mol) Anilin und 239,97 g (1,68 mol) 1-Naphthylamin vor. Zu diesem Gemisch gab man 10,37 g einer 21%igen Lösung von Ammoniumtetrafluoroborat in Wasser. Man erhitzte zunächst unter Normaldruck auf eine Temperatur von ca. 100°C, wobei ca. 5-15 g eines Gemisches aus Anilin und Wasser abdestillierten. Anschließend wurde auf 280°C aufgeheizt, wobei der Druckanstieg hinter dem Kühler mittels Druckhalteventil auf 3 bar begrenzt wurde. Siedendes Anilin wurde durch den Intensivkühler am Entweichen gehindert, während das entstehende Ammoniakgas dem Druckanstieg entsprechend über das Druckhalteventil abgelassen wurde. In regelmäßigen Abständen wurden Proben aus dem Autoklav entnommen und gaschromatographisch analysiert.

| **Laufzeit** | **Anilin** | **1-Naphthylamin** | **N-Phenyl-1-naphthylamin** | **Diphenylamin** | **Umsatz 1-Naphthylamin** | **Ausbeute N-Phenyl-1-naphthylamin** |
|---|---|---|---|---|---|---|
| [min] | [Gew.-%] | [Gew.-%] | [Gew.-%] | [Gew.-%] | [%] | [%] |
| 0 | 50,0 | 45,0 | 1,9 | 0,07 | 0,6 | 2,8 |
| 60 | 40,0 | 27,1 | 24,6 | 0,32 | 40,3 | 35,4 |
| 180 | 31,5 | 11,3 | 49,0 | 0,10 | 75,1 | 70,7 |
| 300 | 28,6 | 5,5 | 59,1 | 0,05 | 87,9 | 85,1 |
| 420 | 27,6 | 4,1 | 61,3 | 0,01 | 91,1 | 88,4 |
| 660 | 26,7 | 2,5 | 61,9 | 0,02 | 94,4 | 89,3 |
| 900 | 26,5 | 2,1 | 64,2 | 0,01 | 95,3 | 92,6 |

### Beispiel 2 (zum Vergleich)

Durchführung wie im Beispiel 1, jedoch ohne Druckhalteventil, bei Normaldruck und bei 210°C .

| **Laufzeit** | **Anilin** | **1-Naphthylamin** | **N-Phenyl-1-naphthylamin** | **Diphenylamin** | **Umsatz 1-Naphthylamin** | **Ausbeute N-Phenyl-1-naphthylamin** |
|---|---|---|---|---|---|---|
| [min] | [Gew.-%] | [Gew.-%] | [Gew.-%] | [Gew.-%] | [%] | [%] |
| 0 | 48,3 | 44,7 | 3,6 | 0,03 | 1,4 | 5,1 |
| 93 | 41,2 | 34,7 | 19,2 | 0,02 | 23,3 | 27,7 |
| 213 | 34,4 | 24,2 | 35,2 | 0,12 | 46,6 | 50,7 |
| 356 | 32,1 | 19,1 | 43,4 | 0,02 | 57,9 | 62,6 |
| 453 | 31,2 | 17,0 | 45,7 | 0,02 | 62,6 | 65,9 |
| 693 | 30,4 | 14,9 | 48,7 | 0,01 | 67,2 | 70,2 |
| 933 | 29,3 | 13,0 | 51,2 | 0,02 | 71,3 | 73,8 |

### Beispiel 3

In einem Hochdruckautoklaven aus V4A, der mit einem Kühler, einem nachgeschalteten Schlangenkühler und dahinter mit einem Druckhalteventil ausgestattet war, wurden 2.550 kg (27,4 kmol) Anilin und 2.180 kg (15,2 kmol) 1-Naphthylamin vorgelegt. Zu dieser Lösung gab man 88 kg einer ca. 21 %igen wäßrigen Katalysatorlösung, die aus Anilin, Borsäure und wäßriger Flußsäure hergestellt worden war. Man erhitzte das Reaktionsgemisch unter Rückfluß innerhalb von 7 Stunden auf 200°C sowie während des weiteren Reaktionsverlaufs innerhalb von 30 Stunden weiter auf 250°C. Die Beheizung erfolgte dabei auf eine derart kontrollierte Weise, daß ständig nur geringe Mengen eines Gemisches aus Ammoniak, Anilin und Wasser über Kopf abdestillierten, während der durch das entstehende Ammoniakgas bedingte Druckanstieg mittels des hinter dem Kühlersystem angebrachten Druckhalteventils auf 2,5 bar_{abs} begrenzt wurde.

Nach 41 Stunden wurde die Umsetzung beendet. Das Reaktionsgemisch hatte nach 31, nach 35 und nach 41 Stunden die nachfolgend aufgelistete, anhand entnommener Proben gaschromatographisch bestimmte Zusammensetzung:

| Stunden | Anilin | 1-NA | PAN | PAN-Aus-beute | DNA | DPA | Umsatz 1-NA |
|---|---|---|---|---|---|---|---|
| | [%] | [%] | [%] | [% d. Th.] | [%] | [%] | [%] |
| 31 | 45,90 | 12,50 | 37,50 | 52,0% | 3,10 | 0,11 | 73,5% |
| 35 | 34,50 | 10,40 | 50,00 | 69,3% | 4,40 | 0,12 | 77,9% |
| 41 | 27,00 | 6,50 | 60,70 | 84,1% | 5,00 | 0,15 | 86,2% |
| PAN = N-Phenyl-1-naphthylamin | | | | | | | |
| 1-NA = 1-Naphthylamin | | | | | | | |
| DNA = Di-(1-naphthylamin) | | | | | | | |
| DPA = Diphenylamin | | | | | | | |

Das Gemisch wurde durch Extraktion und mehrstufige Destillation aufgearbeitet.

## Patentansprüche

1. Verfahren zur diskontinuierlichen oder kontinuierlichen Herstellung von N-Phenyl-1-naphthylamin durch Umsetzung von Anilin und 1-Naphthylamin in kondensierter Phase bei 100-400°C, dadurch gekennzeichnet, daß man die Reaktion in Gegenwart eines Katalysators und bei einem Reaktionsdruck, der oberhalb des Normaldrucks liegt, durchführt.

2. Verfahren nach Anspruch 1, dadurch gekennzeichnet, daß man die Reaktion im Druckbereich oberhalb Normaldruck bis 15 bar, bevorzugt bei 1,5 bis 10 bar, besonders bevorzugt bei 1,5 bis 5 bar, durchführt.

3. Verfahren nach Ansprüchen 1 bis 2, dadurch gekennzeichnet, daß man die Reaktion bei 150 bis 350°C, bevorzugt bei 180 bis 300°C, durchführt.

4. Verfahren nach Ansprüchen 1 bis 3, dadurch gekennzeichnet, daß man die Reaktion in Gegenwart eines durch Umsetzung von Fluorwasserstoff, Borsäure und Anilin und/oder 1-Naphthylamin erhaltenen Katalysatorgemisches durchführt.

5. Verfahren nach Anspruch 4, dadurch gekennzeichnet, daß die katalytisch wirksamen Komponenten des Katalysatorgemisches salzartige Verbindungen der allgemeinen Formel
[A] [B] (II)
sind, in der
A für die Kationen [H]⁺, [NH₄]⁺, [Phenyl-NH₃]⁺, [(1-Naphthyl)-NH₃]⁺, [Phenyl-NH₂-Phenyl]⁺ oder für [Phenyl-NH₂-(1-Naphthyl)]⁺, bevorzugt für die Kationen [H]⁺, [NH₄]⁺, [Phenyl-NH₃]⁺ und [(1-Naphthyl)-NH₃]⁺ und besonders bevorzugt für die Kationen [NH₄]⁺ und [Phenyl-NH₃]⁺ steht und
B für Anionen des Typs [B(OH)ₙF₄₋ₙ]⁻, in der n ganzzahlige Werte von 0 bis 4 annehmen kann, und bevorzugt für das Anion [BF₄]⁻ steht,
wobei die katalytisch wirksamen Komponenten des Katalysatorgemisches bevorzugt die Verbindungen
[NH₄]⁺[BF₄]⁻ und/oder [Phenyl-NH₃]⁺[BF₄]⁻ sind.

6. Verfahren nach Ansprüchen 1 bis 3, dadurch gekennzeichnet, daß man die Reaktion in Gegenwart eines Katalysators aus der Gruppe der Mono-, Di- oder Trisulfonsäuren, bevorzugt der Mono- oder Disulfonsäuren, besonders bevorzugt der Monosulfonsäuren der Formel
R-(SO₃H)ₙ (III)
durchführt, in der
R für geradkettiges oder verzweigtes C₁-C₂₀-Alkyl, oder für unsubstituiertes oder mit bis zu 3 weiteren Substituenten substituiertes Phenyl, Naphthyl oder Pyridinyl steht, wobei als Sustituenten geradkettiges oder verzweigtes C₁-C₆-Alkyl, Halogen, Amino, geradkettiges oder verzweigtes C₁-C₆-Alkylamino oder Di-(C₁-C₆-alkyl)amino, Hydroxy, geradkettig oder verzweigtes C₁-C₆-Alkyloxy oder Carboxyl, bevorzugt geradkettiges oder verzweigtes C₁-C₄-Alkyl, Fluor, Chlor, Brom, Amino oder Hydroxy, besonders bevorzugt Methyl, Chlor, Brom, Amino, Hydroxy oder Carboxyl in Frage kommen, und
n die Zahl 1, 2 oder 3 bedeutet.

7. Verfahren nach Anspruch 6, dadurch gekennzeichnet, daß man die Reaktion in Gegenwart eines Katalysators aus der Gruppe Methan- und Ethansulfonsäure, Benzolsulfonsäure, p-Toluolsulfonsäure, 4-Chlorbenzolsulfonsäure, 4-Brombenzolsulfonsäure, 2-Sulfobenzoesäure, o-, m- und p-Aminobenzolsulfonsäure durchführt.

8. Verfahren nach Ansprüchen 1 bis 7, dadurch gekennzeichnet, daß nach Beendigung der Reaktion eine Extraktion des erhaltenen Produktgemisches mit Wasser oder wasserhaltigen Lösungen zur Abtrennung der katalytisch wirksamen Komponenten durchgeführt wird.

9. Verfahren nach Ansprüchen 1 bis 8, dadurch gekennzeichnet, daß die Reaktionskomponente 1-Naphthylamin oder ein Gemisch von 1-Naphthylamin mit 5-40 % des gesamten Anilins nach Vorlegen des restlichen Anilins und des Katalysators während oder nach Aufheizen auf die gewählte Temperatur und nach Einstellung des gewählten Drucks in das Reaktionsgefäß eindosiert wird.
